# EUROPEAN PATENT SPECIFICATION

(11) **EP 1 905 346 B1**
(45) Date of publication and mention of the grant of the patent: **09.06.2010**
(21) Application number: 07253560.2
(22) Date of filing: 07.09.2007
(51) Int. Cl.: A61B 1/018, A61M 25/09

(54) **Medical drive system for providing motion to at least a portion of a medical apparatus**
Medizinisches Antriebssystem für Bereitstellung von Bewegung für mindestens einen Teil einer medizinischen Vorrichtung
Système de commande médical pour fournir un mouvement à au moins une partie d'un appareil médical

(30) Priority: 08.09.2006 US 518606
(43) Date of publication of application: 02.04.2008
(73) Proprietor: Ethicon Endo-Surgery, Inc., Cincinnati, OH 45242 (US)
(72) Inventor: Bakos, Gregory J., Mason Ohio 45040 (US); Spivey, James T., Loveland Ohio 45140 (US); Long, Gary L., Cincinnati Ohio 45227 (US)
(74) Representative: Tunstall, Christopher Stephen

(56) References cited:
- WO-A-03/053505
- JP-A- 56 008 028
- US-A1- 2005 250 989
- US-A1- 2005 256 429
- US-B1- 6 171 234
- US-B1- 6 554 793

## Description

**Field of the Invention**

The present invention is related generally to medical equipment, and more particularly to a medical drive system for providing motion to at least a portion of a medical apparatus.

**Background of the Invention**

A physician typically accesses and visualizes tissue within a patient's gastrointestinal (GI) tract with a long, flexible endoscope. For the upper GI, a physician may insert a gastroscope into the sedated patient's mouth to examine and treat tissue in the esophagus, stomach, and proximal duodenum. For the lower GI, a physician may insert a colonoscope through the sedated patient's anus to examine the rectum and colon. Some endoscopes have a working channel, typically about 2.5-3.5 millimeters in diameter, extending from a port in the handpiece to the distal portion of the flexible insertion tube. A physician may insert medical devices into the working channel to help diagnose or treat tissues within the patient. Physicians commonly take tissue biopsies from the mucosal lining of the GI tract using a flexible, biopsy forceps through the working channel of the endoscope.

Insertion of a flexible endoscope, especially into the colon, can be a very time-consuming and uncomfortable procedure for the patient, even when sedated with drugs. A physician often needs several minutes to push a flexible endoscope through the convoluted sigmoid, descending, transverse, and ascending portions of the colon. The physician may diagnose and/or treat tissues within the colon either during insertion or removal of the endoscope. The flexible endoscope may "loop" within the colon, such as at the sigmoid colon or at the splenic flexure of the colon, so that it becomes difficult to further advance the endoscope along the colon. When a loop is formed, the force exerted to push the scope stretches the mesentery and causes pain for the patient. Depending on the anatomy of the patient and the skill of the physician in manipulating the flexible endoscope, some portions of the colon may be unexamined, thus increasing the risk of undiagnosed disease.

Guidewires have been used to aid the introduction of catheters and other instruments into many sites in the human body. Many medical applications and specific designs of guidewires have been for cardiovascular use. There are, however, specific challenges relates to the use of guidewires in the GI tract, as opposed to the vascular system. Thus, the bowel is more tortuous, softer and generally of larger diameter. Furthermore, in the case of the small intestine and the colon, these are longer than most arteries or veins.

Documents JP-A-56008028 and US-B-6171234 disclose mechanical drive systems that are suitable to surround a flexible catheter and are adapted to engage and move a medical apparatus inside the flexible catheter.

**Summary**

The invention is a medical drive system which includes a mechanized drive system assembly as claimed in claim 1. The drive system assembly is adapted to engagingly slide onto and surround a flexible catheter. The catheter has a distal end insertable into a body lumen of a patient. The drive system assembly is adapted to engage and move at least a portion of a medical apparatus. Preferred embodiments are disclosed in the dependent claims.

The drive system assembly is adapted to surroundingly attach to, and detach from, a flexible catheter having a distal end insertable into a body lumen of a patient. The drive system assembly is adapted to engage and move at least a portion of a medical apparatus.

The medical drive system can include a spur gear and a nut gear. The spur gear is adapted to be rotatably driven by the drive shaft. The nut gear includes external teeth which are engaged by the spur gear and includes an internal feature adapted to engage external surface features of a medical apparatus to move at least a portion of the medical apparatus.

The drive system assembly can include a rotatable and flexible drive shaft and a nut gear fixedly attached to the drive shaft. The nut gear has internal threads, and the drive shaft has at least a hollow portion. The internal threads are adapted to operatively engage external threads of a medical apparatus when the medical apparatus is positioned in the hollow portion to move at least a portion of the positioned medical apparatus through the nut gear when the nut gear is rotated by the drive shaft.

Several benefits and advantages are obtained from one or more of the expressions of embodiments of the invention. When the medical apparatus is a medical guidewire (or, for example, a medical needle), having a mechanized drive system assembly allows for improved guidewire (or needle) extension, as can be appreciated by those skilled in the art. When the drive system assembly is adapted to slide onto a catheter, existing catheters can be used with the drive system assembly instead of having to manufacture a specialized catheter containing the drive system assembly.

**Brief Description of the Figures**

FIGURE 1 is a perspective view of a first embodiment of a medical drive system of the invention and of a distal end portion of a flexible catheter, wherein the medical drive system has been slid onto and surrounds the catheter, wherein the medical drive system moves a first embodiment of a medical apparatus which includes a medical instrument in the form of a medical-treatment ultrasound transducer assembly, and wherein the top cover of the medical drive system has been removed;

FIGURE 2 is a view of the medical drive system of Figure 1, wherein the medical drive system has been removed off the catheter and the medical apparatus has been removed from the medical drive system;

FIGURE 3 is a side cross sectional view of the nut gear of the medical drive system of Figures 1-2 showing the internal threads of the nut gear which are adapted to operatively engage the external threads of the medical-treatment ultrasound transducer assembly of Figure 1;

FIGURE 4 is a side-elevational view of a portion of a second embodiment of the medical apparatus which includes a medical guidewire and which can be moved by the medical drive system of Figures 1-2;

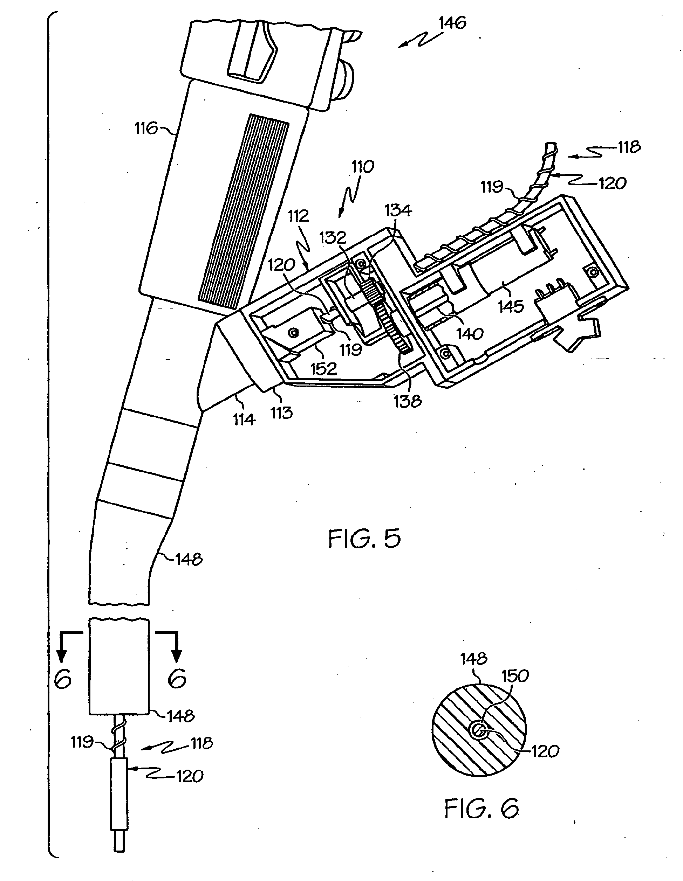

FIGURE 5 is a perspective view of an example of a medical drive system not according to the invention, of a proximal portion of an endoscope having a working channel entrance, of a distal end portion of the endoscope insertion tube, and of an embodiment of a motor assembly (schematically shown with a portion cutaway exposing the drive shaft) operatively attached to the medical drive system, wherein the medical drive system has been slid onto and surrounds the working channel entrance, wherein the medical drive system moves a first embodiment of a medical apparatus which is insertable into the working channel and which includes a medical instrument in the form of a medical-treatment ultrasound transducer assembly, and wherein the side covers of the medical drive system and the motor assembly have been removed;

FIGURE 6 is a cross sectional view of the insertion tube of Figure 5 taken along lines 6-6 of Figure 5;

FIGURE 7 is a view of the medical drive system of Figure 5, wherein the medical drive system has been removed off the working channel entrance and the medical apparatus has been removed from the medical drive system;

FIGURE 8 is a side cross sectional view of the nut gear of the medical drive system of Figures 5 and 7 showing the internal threads of the nut gear which are adapted to operatively engage the external threads of the medical-treatment ultrasound transducer assembly of Figure 5;

FIGURE 9 is a side-elevational view of a portion of a second embodiment of the medical apparatus which includes a medical guidewire and which can be moved by the medical drive system of Figures 5 and 7;

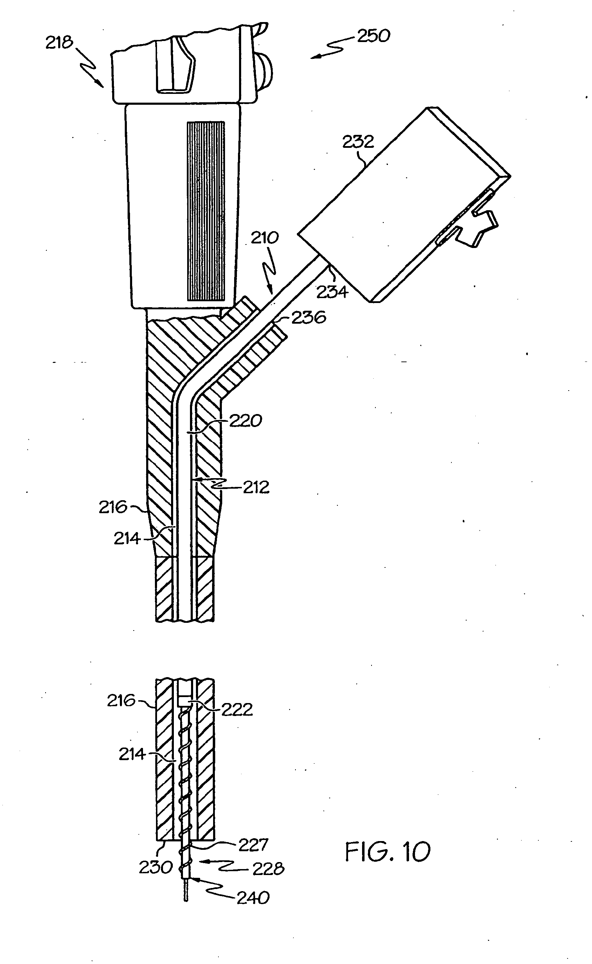

FIGURE 10 a schematic side elevational view of an example of a medical drive system not according to the invention, of a proximal portion of an endoscope shown partially in cross section and having a flexible insertion tube including a working channel, of a distal end portion of the endoscope insertion tube shown in cross section, and of an embodiment of a motor assembly operatively attached to the medical drive system, wherein the drive system assembly has been inserted into the working channel, wherein the sheath of the drive system assembly has been omitted for clarity, and wherein the medical drive system moves a first embodiment of a medical apparatus which includes a medical instrument (shown in exterior view) in the form of a medical-treatment ultrasound transducer assembly;

FIGURE 11 is a view of the medical drive system and the medical instrument of Figure 10 removed from the working channel, wherein the medical drive system, but not the medical instrument, is shown in cross section;

FIGURE 12 is a view of the medical drive system of Figure 11 with the addition of the sheath and with the medical instrument removed;

FIGURE 13 is a cross sectional view of the sheath and the drive shaft of Figure 12 taken along lines 13-13 of Figure 12, and

FIGURE 14 is a side-elevational view of a portion of a second embodiment of the medical apparatus which includes a medical guidewire and which can be moved by the medical drive system of Figures 10-13.

**Detailed Description**

Before explaining the several embodiments of the present invention in detail, it should be noted that each embodiment is not limited in its application or use to the details of construction and arrangement of parts and steps illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is further understood that any one or more of the following-described expressions, embodiments, examples, etc. can be combined with any one or more of the other following-described expressions, embodiments, examples, etc.

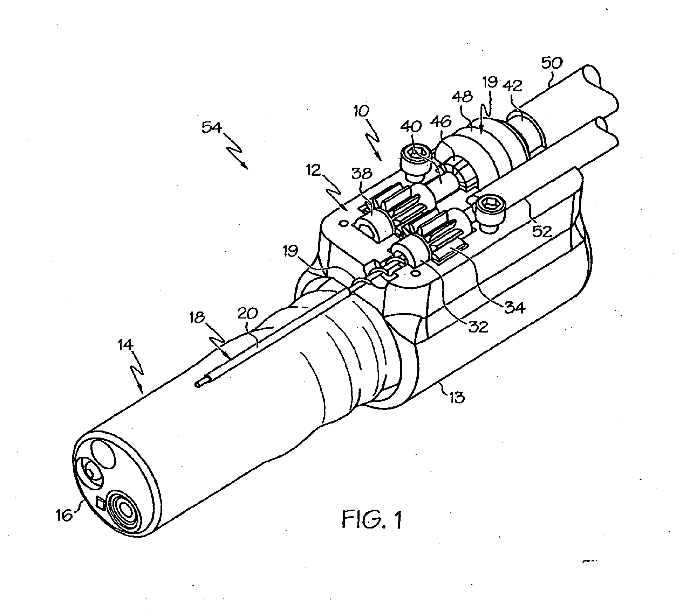

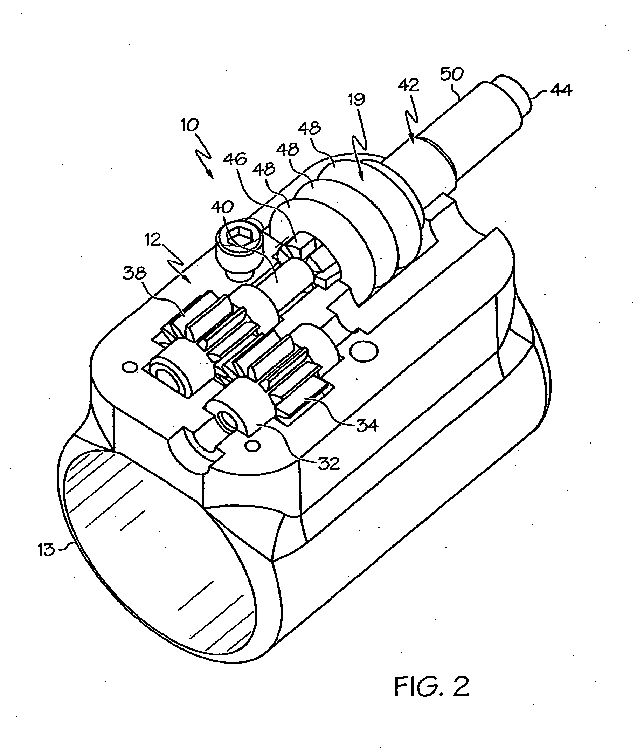

A first embodiment of a medical drive system 10 of the invention is shown in Figures 1-3. A first expression of the medical drive system 10 of Figures 1-3 includes a mechanized drive system assembly 12. The drive system assembly 12 is adapted to engagingly slide onto and surround a flexible catheter 14. The catheter 14 has a distal end 16 insertable into a body lumen of a patient. The drive system assembly 12 is adapted to engage and move at least a portion of a medical apparatus 18.

Examples of catheters include, without limitation, cardio-vascular catheters, pulmonary catheters, and insertion tubes of endoscopes such as insertion tubes of gastroscopes and colonoscopes. Examples of body lumens of a patient include, without limitation, the upper GI (gastrointestinal) tract, the lower GI tract, and blood vessel passageways. Other examples of catheters and/or body lumens are left to the artisan. In one illustration, the drive system assembly 12 includes an elastomeric sleeve portion 13 for surrounding sliding attachment onto, and sliding detachment from, the catheter 14.

In one application of the first expression of the embodiment of Figures 1-3, the drive system assembly 12 is adapted to be disposed proximate the distal end 16 of the catheter 14. In one variation, the catheter 14 is an endoscope insertion tube. In one variation, the drive system assembly 12 is adapted to be disposed just proximal of the articulation portion of an articulatable endoscope insertion tube.

In one design of the first expression of the embodiment of Figures 1-3, the drive system assembly 12 includes a slip clutch 19 which interrupts the mechanized drive system assembly movement of the medical apparatus when the moving medical apparatus encounters resistance over a prescribed threshold.

In one arrangement of the first expression of the embodiment of Figures 1-3, the drive system assembly 12 is adapted to linearly move at least the portion of the medical apparatus 18 beyond the distal end 16 of the catheter 14, wherein the medical apparatus 18 has external threads 19. In another arrangement, not shown, the drive system assembly is adapted to rotatable move at least the portion of the medical apparatus. Other types of movements, including combining linear and rotatable movements, are left to those skilled in the art.

In one employment of the first expression of the embodiment of Figures 1-3, the medical apparatus 18 includes a medical instrument 20 adapted for medically treating patient tissue and having a portion with the external threads 19. In one example, the medical instrument 20 is a medical-treatment ultrasound transducer assembly. Other examples of medical instruments include, without limitation, a forceps assembly, an injection needle, a medical snare, a needle knife, and a sphincterotome. Additional examples are left to the artisan.

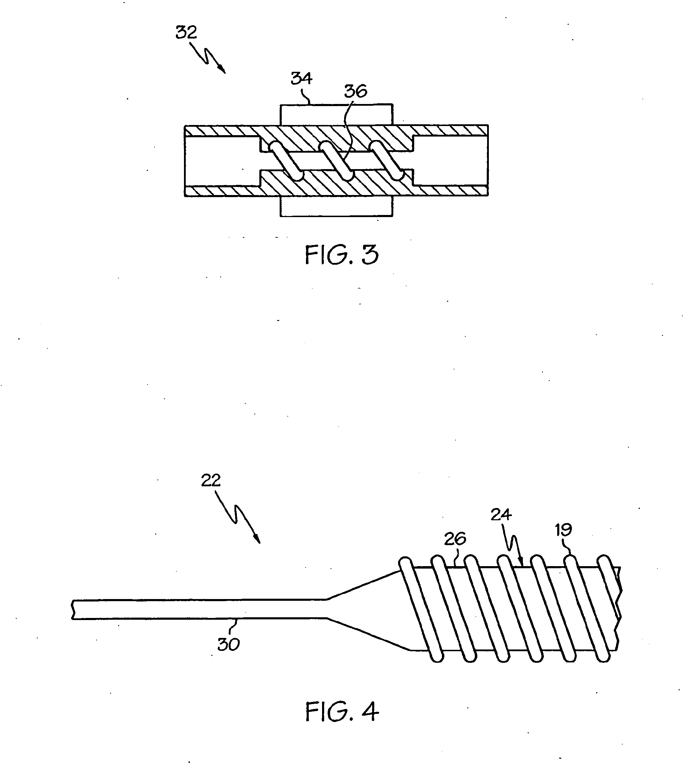

A second embodiment of the medical apparatus 22 suitable for use with the drive system assembly 12 is shown in Figure 4, wherein the medical apparatus 22 includes a medical guidewire 24 having a first segment 26 with the external threads 19. In one variation, the medical guidewire 24 has a distal second segment 30 without external threads. In one modification, not shown, the second segment is covered by an attached lubricious sleeve which creates a low friction surface for easy passage through the body lumen. In one example, not shown, the medical guidewire is a loop-track medical guidewire, wherein the distal end of the second segment is positioned outside the patient. In one choice of materials, the medical guidewire 24, other than any lubricious sleeve, consists essentially of a super-elastic alloy such as nitinol available from Nitinol Devices & Components (Fremont, CA) and the lubricious sleeve Examples of materials for the lubricious sleeve 28 consists essentially of Polytetrafluoroethylene (PTFE), such as Striped Teflon^{®} PTFE available from Zeus, Inc (Orangeburg, SC). In one enablement, the lubricious sleeve is applied through a heat-shrink process well known in the art. In one method of construction, a nitinol helical spring is soldered or laser welded onto a nitinol wire. In another method, a larger diameter nitinol wire is machined to create the external threads.

In one construction of the first expression of the embodiment of Figures 1-3, the drive system assembly 12 includes a nut gear 32 having driven external teeth 34 and having driving internal threads 36 adapted to operatively engage the external threads 19 of the medical apparatus 18 or 22. In one variation, the drive system assembly 12 also includes a spur gear 38 disposed to engage the external teeth 34 of the nut gear 32. In one modification, the slip clutch 19 includes a hexagonal shaft 40 and a coupler 42. The distal end of the hexagonal shaft 40 is connected to the spur gear 38. The coupler 42 includes a proximal end which is attachable to a motor (or hand crank) driven flexible drive shaft 44, includes a distal end in the form of fingers 46 which surround the proximal end of the hexagonal shaft 40, and includes at least one O-ring 48 which surrounds the fingers 46. When the moving medical apparatus 18 or 22 encounters a threshold resistance, the hexagonal shaft 40 stops rotating and the fingers 46 slip over the corners of the hexagonal shaft 40. When the resistance encountered by the medical apparatus 18 or 22 falls below the threshold resistance, the rotating fingers 46 stay on the flats of the hexagonal shaft 40. In one illustration, a first sheath 50 surrounds the drive shaft 44, and a second sheath 52 surrounds the medical apparatus 18 proximal the drive system assembly 12. When the advancing medical apparatus 18 or 22 begins to encounter resistance over a threshold, the drive system assembly 12 will interrupt the mechanized rotation of the medical apparatus 18 or 22 to effectively limit the amount of force it is able to apply to tissue as it is advanced. In one example, the threshold resistance is in the range of 2.5 pounds to 3.5 pounds. Other design examples of slip clutches for rotational movement and design examples of slip clutches for linear movement are left to the artisan.

Other constructions, not shown, include the medical apparatus having surface elevation features other than external threads such as periodic teeth, periodic holes, and/or periodic grooves which are operatively engaged by the drive system assembly. Constructions without surface elevation features on the medical apparatus are left to those skilled in the art.

A second expression of the embodiment of Figures 1-3 is for a medical drive system 10 which includes a mechanized drive system assembly 12. The drive system assembly 12 is adapted to surroundingly attach to, and detach from, a flexible catheter 14 having a distal end 16 insertable into a body lumen of a patient. The drive system assembly 12 is adapted to engage and move at least a portion of a medical apparatus 18.

In one design of the second expression of the embodiment of Figures 1-3, the drive system assembly 12 includes a slip clutch 19 which interrupts the mechanized drive system assembly movement of the medical apparatus when the moving medical apparatus encounters resistance over a prescribed threshold.

In one illustration, not shown, the drive system assembly includes two sleeve sections which are machine screwed together about the catheter.

A third expression of the embodiment of Figures 1-3 is for a medical assembly 54 which includes a flexible catheter 14, a medical drive system 10, and a medical apparatus 18. The medical drive system 10 includes a mechanized drive system assembly 12. The drive system assembly 12 surrounds and is attached to the catheter 14. The catheter 14 has a distal end 16 insertable into a body lumen of a patient. The drive system assembly 12 engages, and is adapted to move, at least a portion of the medical apparatus 18.

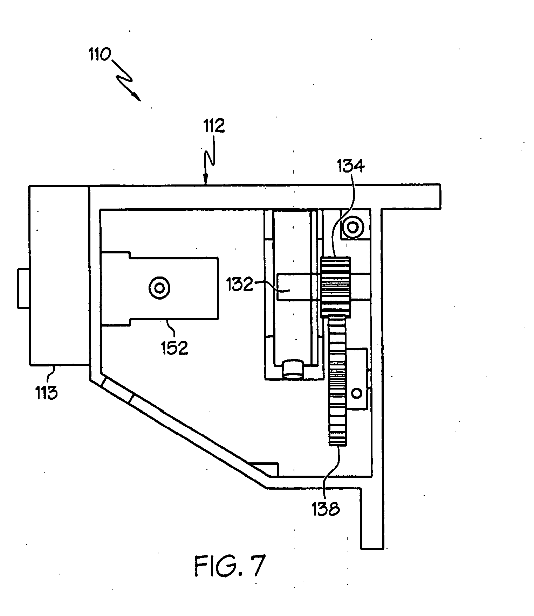

An example of a medical drive system 110 is shown in Figures 5-8. A first expression of the medical drive system 110 of Figures 5-8 includes a mechanized drive system assembly 112. The drive system assembly 112 is adapted to engagingly slide onto and surround a working channel entrance 114 of an endoscope 116. The drive system assembly 112 is adapted to engage and move at least a portion of a medical apparatus 118 wherein the medical apparatus 118 is insertable into the working channel entrance 114.

In one illustration, the drive system assembly 112 includes an elastomeric sleeve portion 113 for surrounding sliding attachment onto, and sliding detachment from, the working channel entrance 114. Other illustrations, such as a clamp attachment, are left to those skilled in the art. In one design of the first expression of the embodiment of Figures 5-8, not shown, the drive system assembly includes a slip clutch which interrupts the mechanized drive system assembly movement of the medical apparatus when the moving medical apparatus encounters resistance over a prescribed threshold.

In one arrangement of the first expression of Figures 5-8, the drive system assembly 112 is adapted to linearly move at least the portion of the medical apparatus 118, wherein the medical apparatus 118 has external threads 119. In another arrangement, not shown, the drive system assembly is adapted to rotatable move at least the portion of the medical apparatus. Other types of movements, including combining linear and rotatable movements, are left to those skilled in the art.

In one employment of the first expression of Figures 5-8, the medical apparatus 118 includes a medical instrument 120 adapted for medically treating patient tissue and having a portion with the external threads 119. In one example, the medical instrument 120 is a medical-treatment ultrasound transducer assembly. Other examples of medical instruments are left to the artisan.

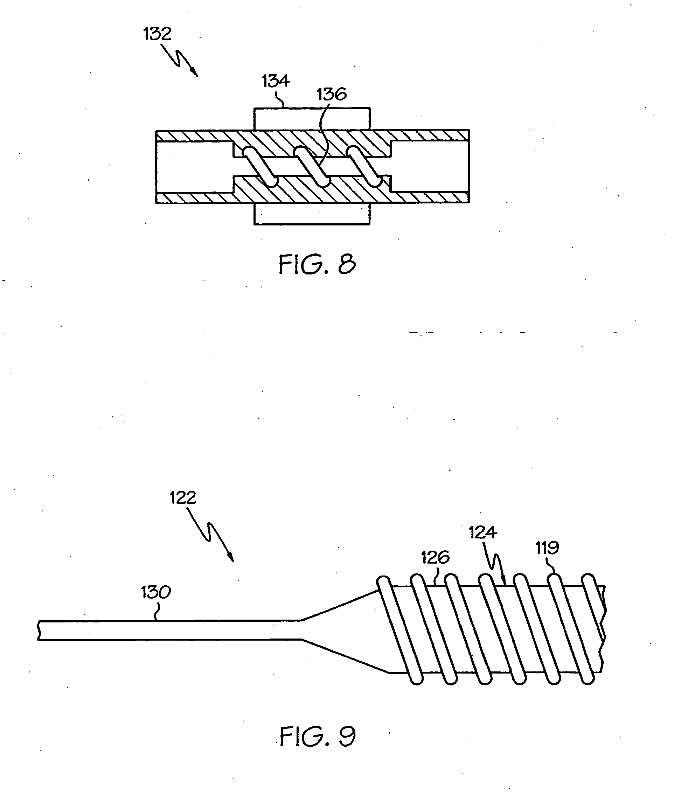

A second example of the medical apparatus 122 suitable for use with the drive system assembly 112 is shown in Figure 9, wherein the medical apparatus 122 includes a medical guidewire 124 having a first segment 126 with the external threads 119. In one variation, the medical guidewire 124 has a distal second segment 130 without external threads.

In one construction of the first expression of Figures 5-8, the drive system assembly 112 includes a nut gear 132 having driven external teeth 134 and having driving internal threads 136 adapted to operatively engage the external threads 119 of the medical apparatus 118. In one variation, the drive system assembly 112 also includes a spur gear 138 disposed to engage the external teeth 134 of the nut gear 132.

A second expression of Figures 5-8 is for a medical drive system 110 which includes a mechanized drive system assembly 112. The drive system assembly 112 includes a spur gear 138 and a nut gear 132. The spur gear 138 is adapted to be rotatably driven by a drive shaft 140. The nut gear 132 includes external teeth 134 which are engaged by the spur gear 138 and includes an internal feature (such as internal threads 136) adapted to engage external surface features (such as external threads 119) of a medical apparatus 118 to move at least a portion of the medical apparatus 118.

In one illustration, a motor 145 is used to rotate the drive shaft 140. In another illustration, not shown, a hand crank is used to rotate the drive shaft. Other devices for rotating the drive shaft are left to the artisan. In one design of the first expression of the embodiment of Figures 5-8, not shown, the drive system assembly includes a slip clutch which interrupts the mechanized drive system assembly movement of the medical apparatus when the moving medical apparatus encounters resistance over a prescribed threshold.

In one employment of the second expression of Figures 5-8, the medical apparatus 118 includes a medical instrument 120 adapted for medically treating patient tissue. In one example, the medical instrument 120 is a medical-treatment ultrasound transducer assembly. In one procedure, the medical drive system 112 is not used in association with an endoscope. In one variation, the medical drive system 112 is not used with any other medical appliance having a catheter.

A third expression of Figures 5-8 is for a medical assembly 146 which includes an endoscope 116 having a working channel entrance 114, a medical drive system 110, and a medical apparatus 118. The medical apparatus 118 is inserted into the working channel entrance 114. The medical drive system 110 includes a mechanized drive system assembly 112 which surrounds and is attached to the working channel entrance 114. The drive system assembly 112 engages, and is adapted to move, at least a portion of the medical apparatus 118. It is noted that Figure 5 also shows the flexible insertion tube 148 of the endoscope 116, and Figure 6 shows the working channel 150 of the insertion tube 148 with a portion of the medical instrument 120 disposed therein. It is also noted that a guide 152 for the medical instrument 120 is shown in the drive system assembly 112 of Figure 5.

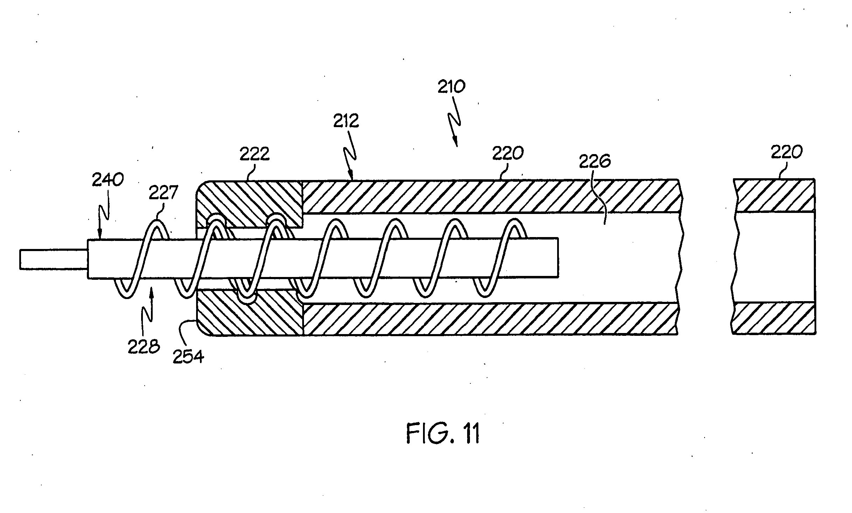

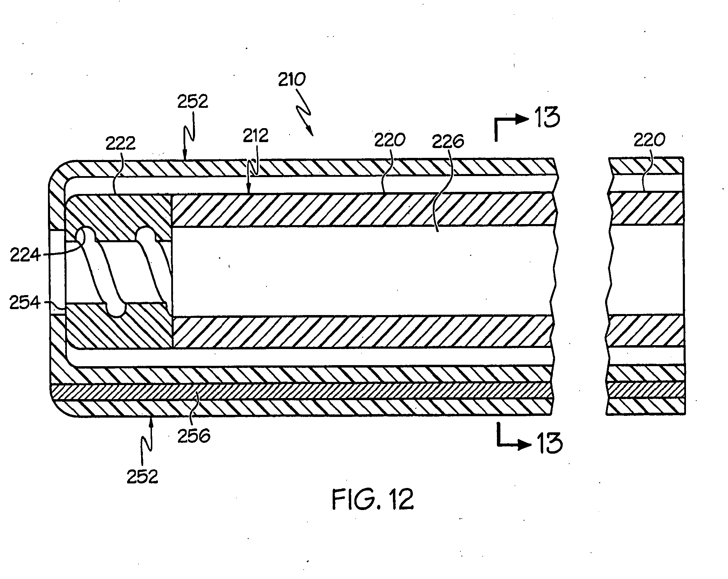

A third example of a medical drive system 210 is shown in Figures 10-13. A first expression of the medical drive system 210 of Figures 10-13 includes a mechanized drive system assembly 212. The drive system assembly 212 is insertable into and withdrawable from a working channel 214 of a flexible insertion tube 216 of an endoscope 218. The drive system assembly 212 includes a rotatable and flexible drive shaft 220 and a nut gear 222 fixedly attached to the drive shaft 220. The nut gear 222 has internal threads 224, and the drive shaft 220 has at least a hollow portion 226. The internal threads 224 are adapted to operatively engage external threads 227 of a medical apparatus 228 when the medical apparatus 228 is positioned in the hollow portion 226 to move at least a portion of the positioned medical apparatus 228 through the nut gear 222 and extendingly beyond a distal end 230 of the insertion tube 216 when the nut gear 222 is rotated by the drive shaft 220.

In one illustration, a motor assembly 232 is disposed outside the endoscope 218 and the patient and is operatively connected to the proximal end 234 of the drive shaft 220. It is noted that the working channel 214 extends from the working channel entrance 236 of the endoscope 218 to the distal end 230 of the insertion tube 216. In another illustration, not shown, a hand crank is used to rotate the drive shaft. Other devices for rotating the drive shaft are left to the artisan.

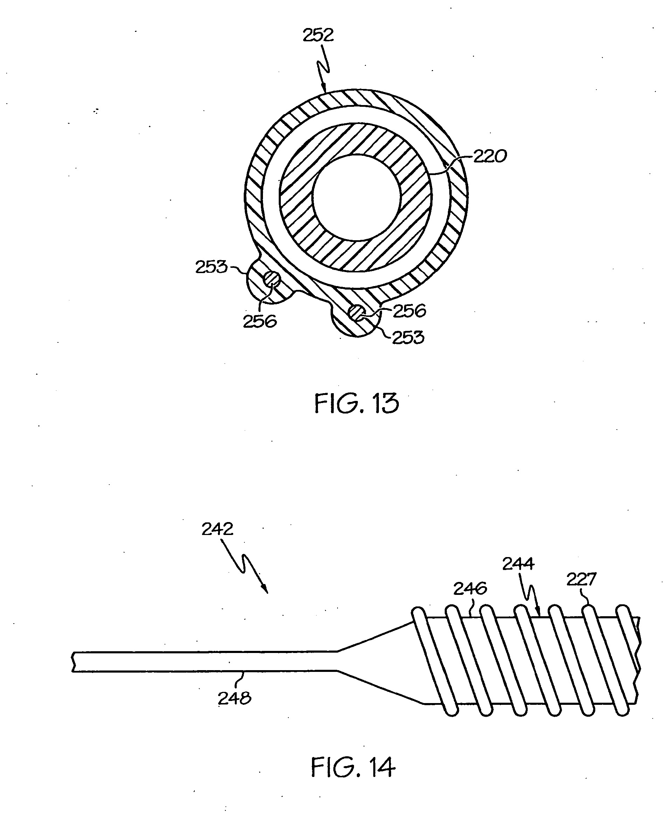

In one construction of the first expression of Figures 10-13, the drive system assembly 212 includes a sheath 252 surrounding the drive shaft 220, wherein the drive shaft 220 is rotatable with respect to the sheath 252 and wherein the sheath 252 includes a plurality of radially outward protrusions 253. In one variation, the sheath 252 is a multi-lumen sheath and extends from the proximal end 234 of the drive shaft 220 to past the distal end 254 of the nut gear 222. In this variation, the sheath 252 radially overlaps the distal end 254 of the nut gear 222, and non-stretch wire 256, such as nitinol wire, is tightly disposed in the smaller lumens of the sheath 252 and extends the length of the sheath 252 to prevent elongation of the drive shaft 220, as can be appreciated by those skilled in the art. It is noted that when the sheath 252 is inserted into the working channel 214, partitioned space still exists around the sheath 252 (such as the spaces between and to the outer sides of the "mouse ears" of the sheath 252 of Figure 13) so that fluids and small devices may pass within the working channel 214 without being disturbed by the rotating flexible drive shaft 220.

In one employment of the first expression of Figures 10-13, the medical apparatus 228 includes a medical instrument 240 adapted for medically treating patient tissue and having a portion with the external threads 227. In one example, the medical instrument 240 is a medical-treatment ultrasound transducer assembly. Other examples of medical instruments are left to the artisan.

A second example of the medical apparatus 242 suitable for use with the drive system assembly 212 is shown in Figure 14, wherein the medical apparatus 242 includes a medical guidewire 244 having a first segment 246 with the external threads 227. In one variation, the medical guidewire 244 has a distal second segment 248 without external threads.

Applicants have experimentally found that a relatively loose engagement of the internal and external threads 224 and 227 results in translation of the medical apparatus 228 without substantial rotation of the medical apparatus 228. In one design, not shown, the proximal end of the medical apparatus 228 has a keyed surface feature which allows the medical apparatus to translate, but not rotate, within a matching lumen of a handle. Other non-rotation techniques are left to those skilled in the art.

In one arrangement of the first expression of Figures 10-13, not shown, the nut gear is a two-piece nut gear attached to a gear housing which is attached to the distal end of the drive shaft. In this arrangement, the proximal end of a tubing is rotatably attached to the nut gear housing, and a resilient end gripper is fixedly attached to the distal end of the tubing. When the assemblage is fully inserted into the working channel, the distal end of the end gripper is located proximate the distal end of the insertion tube and the proximal end of the tubing is located proximate the proximal end of the articulatable section of the insertion tube.

A second expression of Figures 10-13 is for a medical drive system 210 which includes a mechanized drive system assembly 212. The drive system assembly 212 includes a rotatable and flexible drive shaft 220 and a nut gear 222 fixedly attached to the drive shaft 220. The nut gear 222 has internal threads 224, and the drive shaft 220 has at least a hollow portion 226. The internal threads 224 are adapted to operatively engage external threads 227 of a medical apparatus 228 when the medical apparatus 228 is positioned in the hollow portion 226 to move at least a portion of the positioned medical apparatus 228 through the nut gear 222 when the nut gear 222 is rotated by the drive shaft 220.

In one construction of the second expression of Figures 10-13, the drive system assembly 212 includes a sheath 252 surrounding the drive shaft 220, wherein the drive shaft 220 is rotatable with respect to the sheath 252.

In one employment of the second expression of Figures 10-13, the medical apparatus 228 includes a medical instrument 240 adapted for medically treating patient tissue and having a portion with the external threads 227. In one example, the medical instrument 240 is a medical-treatment ultrasound transducer assembly. In one procedure, the medical drive system 210 is not used in association with an endoscope. In one variation, the medical drive system 210 is not used or with any other medical appliance having a catheter.

A third expression of the example of Figures 10-13 is for a medical assembly 250 which includes an endoscope 218 having a flexible insertion tube 216 a working channel 214, a medical drive system 210, and a medical apparatus 228. The medical drive system 210 includes a mechanized drive system assembly 212 insertable into and withdrawable from the working channel 214. The drive system assembly 212 includes a rotatable and flexible drive shaft 220 and a nut gear 222 fixedly attached to the drive shaft 220. The nut gear 222 has internal threads 224, and the drive shaft 220 has at least a hollow portion 226. The internal threads 224 operatively engage external threads 227 of a medical apparatus 228 disposed in the hollow portion 226. The internal threads 224 are adapted move at least a portion of the disposed medical apparatus 228 through the nut gear 222 and extendingly beyond a distal end 230 of the insertion tube 216 when the nut gear 222 is rotated by the drive shaft 220.

Several benefits and advantages are obtained from one or more of the expressions of embodiments of the invention. When the medical apparatus is a medical guidewire (or, for example, a medical needle), having a mechanized drive system assembly allows for improved guidewire (or needle) extension, as can be appreciated by those skilled in the art. When the drive system assembly is adapted to slide onto a catheter, existing catheters can be used with the drive system assembly instead of having to manufacture a specialized catheter containing the drive system assembly. When the drive system assembly is adapted to slide onto a working channel entrance of an endoscope or into a working channel of a flexible insertion tube of an endoscope, the drive system assembly may be removed when the working channel is needed for other endoscopic procedures without removing the insertion tube from the patient.

While the present invention has been illustrated by a description of several expressions, embodiments, and examples, etc. thereof, it is not the intention of the applicants to restrict or limit the scope of the appended claims to such detail. Numerous other variations, changes, and substitutions will occur to those skilled in the art without departing from the scope of the claims. It will be understood that the foregoing description is provided by way of example, and that other modifications may occur to those skilled in the art without departing from the scope of the appended Claims.

## Claims

1. A medical drive system (10) comprising a mechanized drive system assembly (12) which is adapted to engagingly slide onto and surround a flexible catheter (14) having a distal end (16) insertable into a body lumen of a patient and which is adapted to engage and move at least a portion of a medical apparatus (18, 22), wherein the drive system assembly comprises a sleeve portion (13) having an internal surface engagingly surrounding a portion of the catheter when the medical drive system engagingly slides onto the catheter, and an opposite external surface on which the drive system assembly can engage and move the medical apparatus, externally of said flexible catheter (14).

2. The medical drive system of claim 1, wherein the drive system assembly is adapted to be disposed proximate a distal end of the catheter.

3. The medical drive system of claim 2, wherein the catheter is an endoscope insertion tube.

4. The medical drive system of claim 3, wherein the drive system assembly is adapted to linearly move at least the portion of the medical apparatus beyond the distal end uf the catheter, and wherein the medical apparatus has external threads.

5. The medical drive system of claim 4, wherein the medical apparatus (18) includes a medical instrument (20) adapted for medically treating patient tissue.

6. The medical drive system of claim 4, wherein the medical apparatus (22) includes a medical guidewire (24).

7. The medical drive system of claim 4, wherein the drive system assembly includes a nut gear (32) having driven external teeth (34) and having driving internal threads (36) adapted to operatively engage the external threads.

8. The medical drive system of claim 7, wherein the drive system assembly includes a spur gear (38) disposed to enrage the external teeth of the nut gear.

9. The medical drive system of claim 1, wherein the drive system assembly includes a slip clutch which interrupts the mechanized drive system assembly movement of the medical apparatus when the moving medical apparatus encounters resistance over a prescribed threshold.

## Patentansprüche

1. Medizinisches Antriebssystem (10), das eine mechanisierte Antriebssystemanordnung (12) umfasst, die eingerichtet ist, eingreifend auf einen flexiblen Katheter (14) mit einem distalen Ende (16), das in ein Körperlumen eines Patienten einführbar ist, zu gleiten und diesen zu umgeben, und die eingerichtet ist, in mindestens einen Abschnitt einer medizinischen Vorrichtung (18, 22) einzugreifen und diesen zu bewegen, wobei die Antriebssystemanordnung einen Hülsenabschnitt (13) mit einer inneren Oberfläche, die eingreifend einen Abschnitt des Katheters umgibt, wenn das medizinische Antriebssystem eingreifend auf den Katheter gleitet, und einer gegenüberliegenden externen Oberfläche, auf der die Antriebssystemanordnung in die medizinische Vorrichtung eingreifen und diese bewegen kann, extern zu dem flexiblen Katheter (14), umfasst.

2. Medizinisches Antriebssystem nach Anspruch 1, wobei die Antriebssystemanordnung eingerichtet ist, in der Nähe eines distalen Endes des Katheters angeordnet zu werden.

3. Medizinisches Antriebssystem nach Anspruch 2, wobei der Katheter ein Endoskopeinführtubus ist.

4. Medizinisches Antriebssystem nach Anspruch 3, wobei die Antriebssystemanordnung eingerichtet ist, mindestens den Abschnitt der medizinischen Vorrichtung linear über das distale Ende des Katheters hinaus zu bewegen, und wobei die medizinische Vorrichtung externe Gewinde hat.

5. Medizinisches Antriebssystem nach Anspruch 4, wobei die medizinische Vorrichtung (18) ein medizinisches Instrument (20) aufweist, das zur medizinischen Behandlung von Patientengewebe eingerichtet ist.

6. Medizinisches Antriebssystem nach Anspruch 4, wobei die medizinische Vorrichtung (22) einen medizinischen Führungsdraht (24) aufweist.

7. Medizinisches Antriebssystem nach Anspruch 4, wobei die Antriebssystemanordnung ein Muttergetriebe (32) mit angetriebenen externen Zähnen (34) und mit antreibenden internen Gewinden (36) aufweist, die eingerichtet sind, betriebstechnisch in die externen Gewinde einzugreifen.

8. Medizinisches Antriebssystem nach Anspruch 7, wobei die Antriebssystemanordnung ein Stirnzahnrad (38) aufweist, das angeordnet ist, um in die externen Zähne des Muttergetriebes einzugreifen.

9. Medizinisches Antriebssystem nach Anspruch 1, wobei die Antriebssystemanordnung eine Schlupfkupplung aufweist, die eine Bewegung der mechanisierten Antriebssystemanordnung der medizinischen Vorrichtung unterbricht, wenn die sich bewegende medizinische Vorrichtung auf einen Widerstand oberhalb eines vorgeschriebenen Schwellwerts trifft.

## Revendications

1. Système de commande médical (10) comprenant un ensemble système de commande mécanisé (12) qui est adapté pour coulisser par mise en prise sur et entourer un cathéter flexible (14) ayant une extrémité distale (16) insérable dans une lumière corporelle d'un patient et qui est adapté pour se mettre en prise avec et déplacer au moins une partie d'un appareil médical (18, 22), dans lequel l'ensemble système de commande comprend une partie de manchon (13) ayant une surface interne entourant par mise en prise une partie du cathéter lorsque le système de commande médical coulisse par mise en prise sur le cathéter et une surface externe opposée sur laquelle l'ensemble système de commande peut se mettre en prise avec et déplacer l'appareil médical, à l'extérieur dudit cathéter flexible (14).

2. Système de commande médical selon la revendication 1, dans lequel l'ensemble système de commande est adapté pour être disposé près d'une extrémité distale du cathéter.

3. Système de commande médical selon la revendication 2, dans lequel le cathéter est un tube d'insertion d'endoscope.

4. Système de commande médical selon la revendication 3, dans lequel l'ensemble système de commande est adapté pour déplacer linéairement au moins la partie de l'appareil médical au-delà de l'extrémité distale du cathéter et dans lequel l'appareil médical présente des filets externes.

5. Système de commande médical selon la revendication 4, dans lequel l'appareil médical (18) comprend un instrument médical (20) adapté pour traiter médicalement le tissu du patient.

6. Système de commande médical selon la revendication 4, dans lequel l'appareil médical (22) comprend un fil guide médical (24).

7. Système de commande médical selon la revendication 4, dans lequel l'ensemble système de commande comprend un engrenage à écrou (32) ayant des dents externes entraînées (34) et ayant des filets internes d'entraînement (36) adaptés pour se mettre en prise opérationnellement avec les filets externes.

8. Système de commande médical selon la revendication 7, dans lequel l'ensemble système de commande comprend un engrenage cylindrique (38) disposé pour se mettre en prise avec les dents externes de l'engrenage à écrou.

9. Système de commande médical selon la revendication 1, dans lequel l'ensemble système de commande comprend un embrayage à friction qui interrompt le mouvement de l'ensemble système de commande mécanisé de l'appareil médical lorsque l'appareil médical mobile rencontre une résistance sur un seuil déterminé.
